# EUROPEAN PATENT SPECIFICATION

(11) **EP 1 396 724 B1**
(45) Date of publication and mention of the grant of the patent: **16.08.2006**
(21) Application number: 02733326.9
(22) Date of filing: 05.06.2002
(51) Int. Cl.: G01N 33/531, G01N 33/537

(54) **METHOD OF ASSAY BY IMMUNOREACTION AND REAGENT FOR USE IN THE IMMUNOREACTION ASSAY**
TEST-VERFAHREN DURCH IMMUNOREAKTION UND REAKTIONSMITTEL ZUR VERWENDUNG BEI EINEM IMMUNOREAKTIONSTEST
PROCEDE D'ANALYSE PAR IMMUNOREACTION, ET REACTIF CORRESPONDANT

(30) Priority: 14.06.2001 JP 2001179710; 03.12.2001 JP 2001368286
(43) Date of publication of application: 10.03.2004
(73) Proprietor: MATSUSHITA ELECTRIC INDUSTRIAL CO., LTD., Kadoma-shi, Osaka 571-8501 (JP)
(72) Inventor: KAMEI, Akihito, Yawata-shi, Kyoto 614-8295 (JP); KENJO, Noriko, Hirakata-shi, Osaka 573-0036 (JP); KAWAMURA, Tatsuro, Kyotanabe-shi, Kyoto 610-0351 (JP); HIRAI, Mahito, Kyotanabe-shi, Kyoto 610-0341 (JP)
(74) Representative: Balsters, Robert
(86) International application number: PCT/JP2002/005568
(87) International publication number: WO 2003/010541

(56) References cited:
- EP-A- 1 061 368
- WO-A-91/10911
- JP-A- 2 036 354
- JP-A- 2 061 561
- JP-A- 6 082 450
- JP-A- 7 270 413
- JP-A- 11 344 494
- US-A- 4 672 045
- US-A- 5 616 460

## Description

### TECHNICAL FIELD

The present invention relates to an immune reaction measurement method capable of measuring an antigen or an antibody (subject substances) contained in a sample.

### BACKGROUND ART

In order to diagnose various diseases and examine progression of disease conditions, the level of proteins, which exists in the human body fluid and is characteristic to the diseases, are measured. Such a technique is widely used in the medical field.

Immune reaction measurement methods utilizing highly-specific antigen-antibody reactions have been predominantly used to measure the content of such proteins. At present, various principles have been applied to develop and exploit immune reaction measurement methods.

Among them, measurement methods for detecting an agglutination complex generated by an antigen-antibody reaction, such as nephelometry, turbidimetry, slide agglutination, ana the like, are well known. These methods are performed within a solution in which an antigen and an antibody are uniformly dispersed, and therefore, are collectively called homogeneous immune reaction measurement methods.

In these methods, a reaction system becomes cloudy due to generation agglutination complexes and the cloudiness depends on the amounts of an antigen and an antibody. Nephelometry and turbidimetry are methods of optically measuring the cloudiness. In nephelometry, cloudiness is determined by measuring the amount of light scattered by a reaction system. In turbidimetry, determination of cloudiness is based upon measurement of light transmission reduced by scattering in a reaction system. In general, the same reaction system can be used and measured by the two methods. A subject which can be measured by one method, can also be measured by the other method. Slide agglutination is a method for determining cloudiness caused by generated agglutination complexes by visual observation or the like. Slide agglutination can employ the same reaction system as used for nephelometry and turbidimetry.

In the above-described conventional homogeneous immune reaction measurement methods, various additives have been tested in order to accelerate an antigen-antibody reaction and measure a trace amount of component with high sensitivity. A well known example is a method of improving reaction time and measurement sensitivity by allowing a water-soluble polymer,such aspolyethylene glycol,dextran, polyvinylpyrrolidone, polyvinyl chloride, or the like, to coexist in a reaction system so as to accelerate formation of agglutination complexes due to an antigen-antibody reaction. Among these water-soluble polymers, polyethylene glycol is known to have a high level of effect even at a relatively low concentration. Polyethylene glycol having an average molecular weight of 6,000 is widely used at a concentration of 2 to 6% by weight (hereinafter abbreviated wt%). Particularly, 4 wt% concentration is believed to produce only a small level of non-specific cloudiness, i.e., highly effective.

The acceleration of an antigen-antibody reaction by a water-soluble polymer tends to increase, as the molecular weight or the concentration of the polymer is increased (see Automated Immunoanalysis Part 1, Ritchie ed., PP. 67-112 (1978)).

Concerning measurement of an antigen-antibody reaction, the higher the strength of a signal antigen-antibody reaction depending on the concentration of an antigen, the more satisfactory the S/N ratio, i.e., the stabler the measurement. However, when an attempt is made to obtain the above-described effect by further acceleration of an antigen-antibody reaction, a higher molecular weight or a higher concentration is required in the case of addition of a conventional water-soluble polymer. In this case, however, the viscosity of a solution, in which such a water-soluble polymer is dissolved, is high, thereby making it difficult to handle it during manipulation for analysis.

In homogeneous immune reaction measurement methods, the zone phenomenon is generally known. The zone phenomenon refers to a phenomenon such that when the amount of one of an antigen and an antibody exceeds the equivalent weight region thereof which forms the largest agglutination complex, generation of the agglutination complex is hindered. A binding reaction between a polyvalent antibody and a more than monovalent antigen is explained by the famous Heidelberger's lattice hypothesis, the details of which are described in Fundamental Immunology, William E. Paul Ed, (1984) (Japanese language translation, Kiso Menekigaku, supervised by Tomio Tada, pp. 714-716 (1987)).

In actual homogeneous immune reaction measurements, generally, an antibody is used to measure the concentration of an antigen, and a measurement value (i.e., an antigen concentration) often has a more important meaning when it is high than when it is low. Therefore, a zone phenomenon due to excess antigens often causes problems. In regions other than a zone, a huge molecular chain containing a complex, in which antibodies and antigens are alternately linked together, is generated. The amount or size of the chain is increased depending on the antigen concentration if the antibody concentration is constant. Therefore, by determining the amount or size of the molecular chain by measuring the optical variation thereof, the antigen concentration can be quantitatively determined. Moreover, an antigen-antibody complex can be sufficiently observed even by naked eye as cloudiness or agglutinations in a solution, depending on the concentration of an antibody and an antigen. Therefore, the antigen concentration can be qualitatively determined by visual observation.

However, in an antigen excess region, since an antigen is present in a larger amount than that of an antibody, the amount of the antibody whose binding site is saturated with the antigen is increased. Therefore, generation of a molecular chain as described above is hindered, and the reaction result cannot distinguish a higher antigen concentration from a low antigen concentration. For this reason, correct quantification and determination according to the antigen concentration cannot be performed. Unfortunately, a concentration range to be measured has to be limited in order to avoid such a situation.

An object of the present invention is to solve the above-described conventional problems by providing an immune reaction measurement method capable of easily increasing measurement values. Another object of the present invention is to provide an immune reaction measurement method capable of relaxing a zone phenomenon in an antigen excess region.

### DISCLOSURE OF THE INVENTION

In order to solve the above-described problems an immune reaction measurement method for measuring an antigen or an antibody (subject substances) contained in a sample according to the present invention is characterized in that it comprises a step A of mixing tricarboxylic acid or tricarboxylate, an antibody or an antigen capable of specifically binding to the subject substance (specifically binding substances) and the sample; and a step B of detecting an agglutination complex generated by an antigen-antibody reaction between the subject substance and the specifically binding substance in a reaction system comprising the sample, the specifically binding substance and the tricarboxylic acid or the tricarboxylate composed by step A, and in that the tricarboxylic acid or tricarboxylate concentration of the reaction system is no more than 0.2 M and the pH of the reaction system is 4.5 when the antigen-antibody reaction is carried out.

### BRIEF DESCRIPTION OF THE DRAWINGS

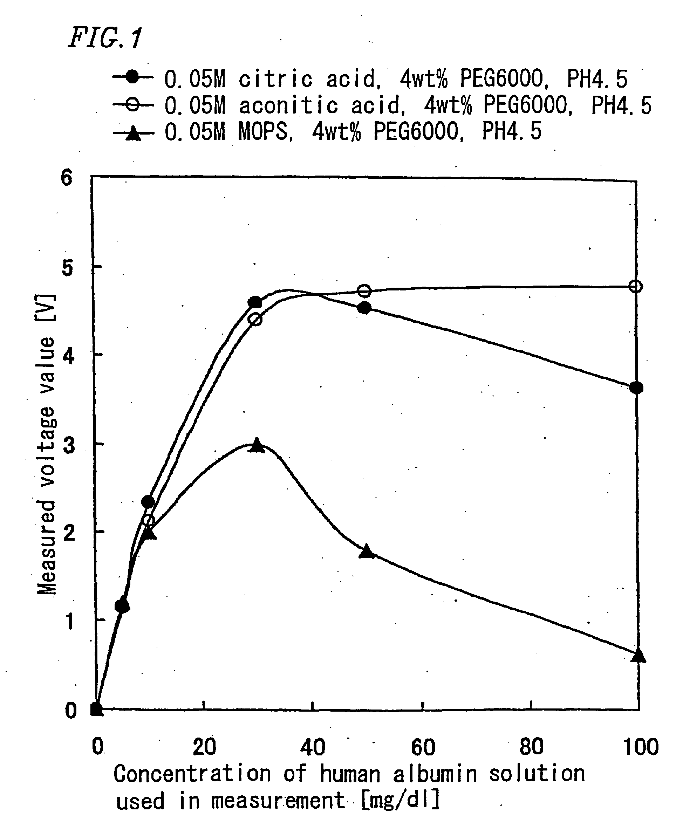

Figure **1** is a graph showing results of measurement of human albumin by immunological nephelometry using an immune reaction measurement method with an immune reaction measurement reagent according to an example of the present invention or according to a comparative example.

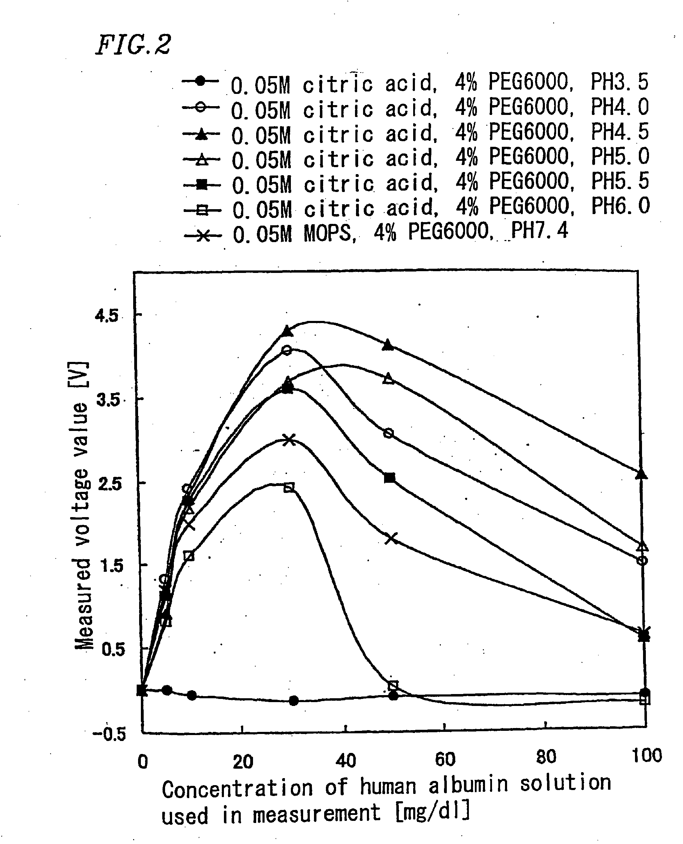

Figure **2** is a graph showing results of investigating the pH dependency of human albumin measurement by immunological nephelometry using an immune reaction measurement method with an immune reaction measurement reagent containing citric acid according to another example of the present invention or according to a comparative example.

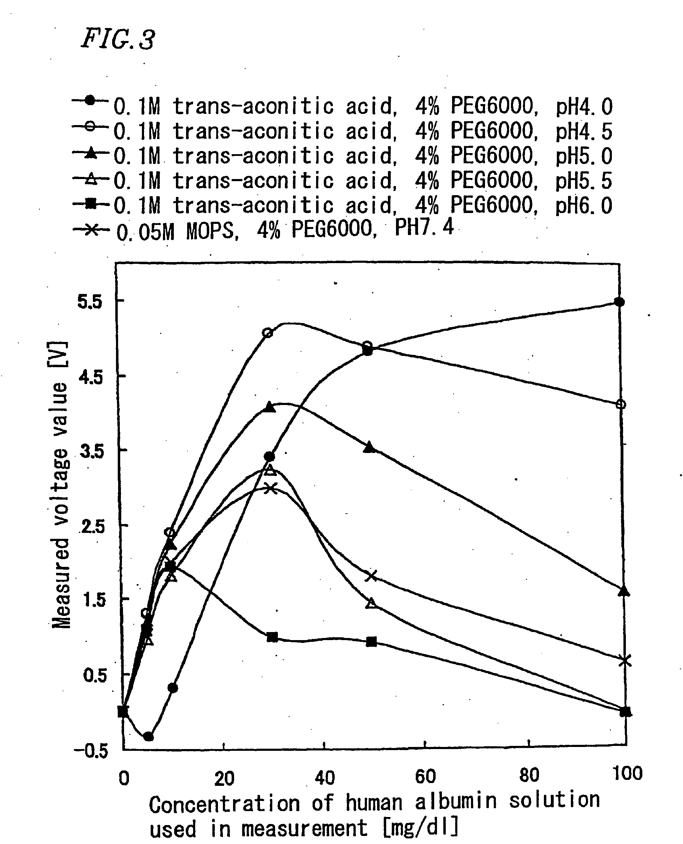

Figure **3** is a graph showing results of investigating the pH dependency of human albumin measurement by immunological nephelometry using an immune reaction measurement method with an immune reaction measurement reagent containing trans-aconitic acid according to the example of the present invention or according to a comparative example.

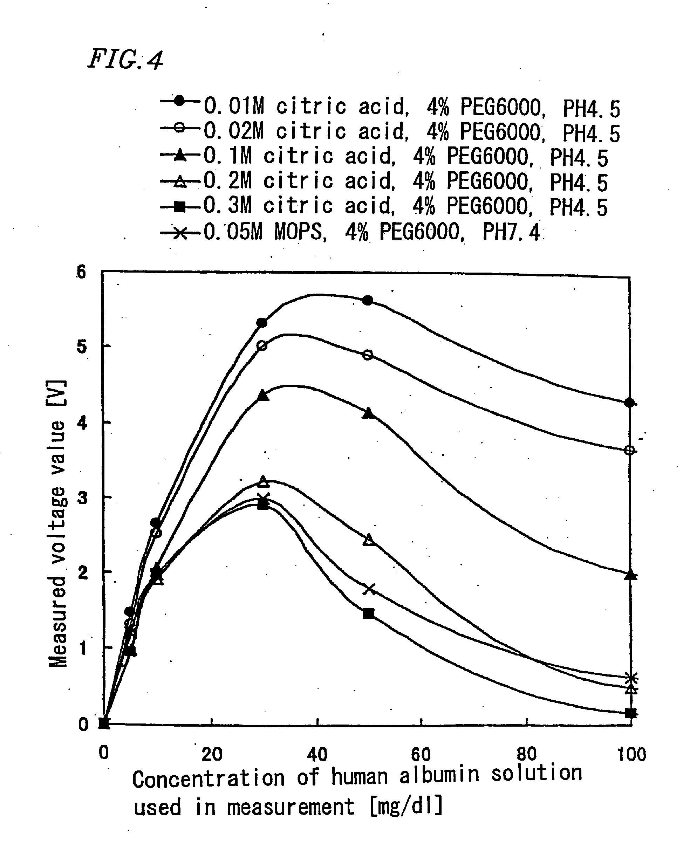

Figure **4** is a graph showing results of investigating the citric acid concentration dependency of human albumin measurement by immunological nephelometry using an immune reaction measurement method with an immune reaction measurement reagent containing citric acid according to still another example of the present invention.

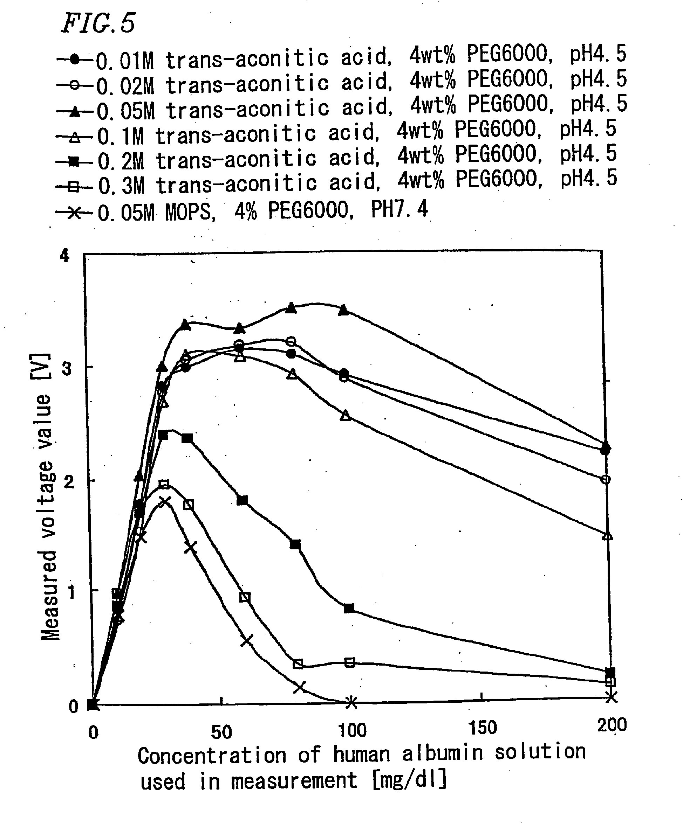

Figure **5** is a graph showing results of investigating the trans-aconitic acid concentration dependency of human albumin measurement by immunological nephelometry using an immune reaction measurement method with an immune reaction measurement reagent containing trans-aconitic acid according to the example of the present invention.

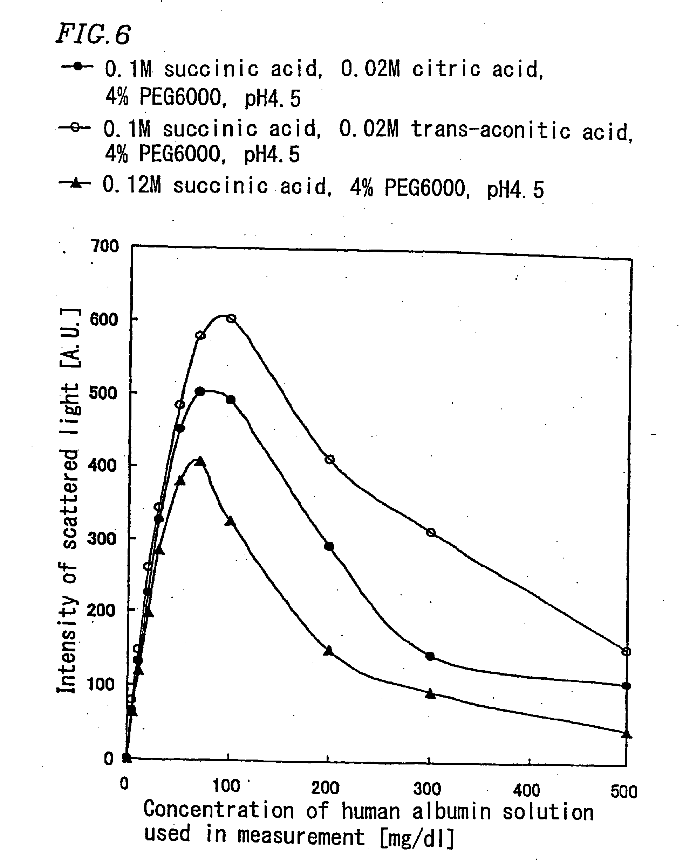

Figure **6** is a graph showing results of measurement of human albumin by immunological nephelometry using an immune reaction measurement method with an immune reaction measurement reagent containing tricarboxylic acid or tricarboxylate and another buffer according to another example of the present invention or according to a comparative example.

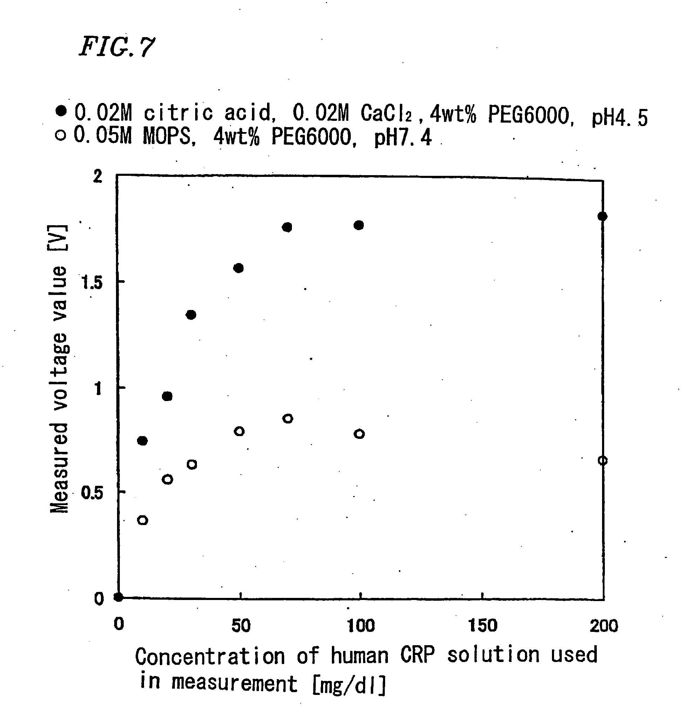

Figure **7** is a graph showing results of measurement of human CRP by immunological nephelometry using an immune reaction measurement method with an immune reaction measurement reagent containing a goat anti-human CRP polyclonal antibody and citric acid according to another example of the present invention or according to a comparative example.

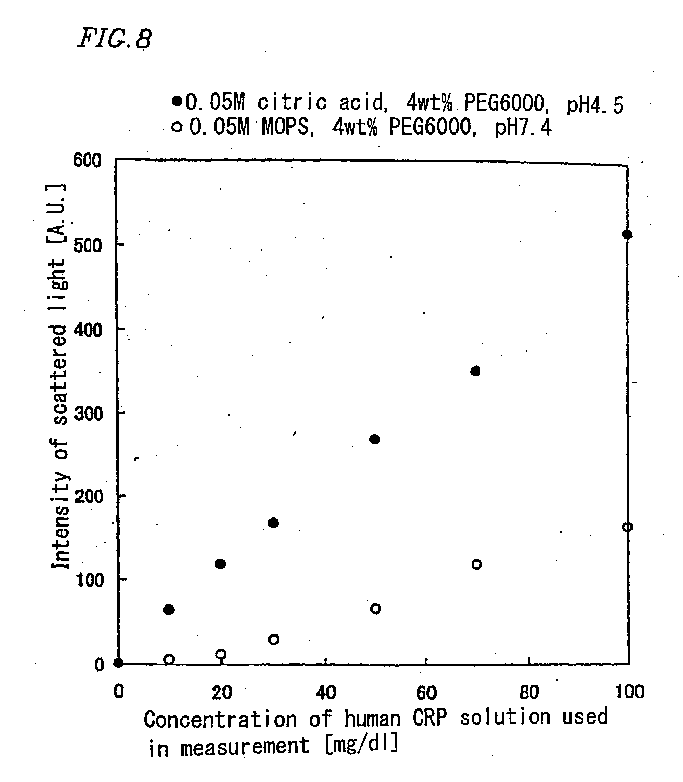

Figure **8** is a graph showing results of measurement of human CRP by immunological nephelometry using an immune reaction measurement method with an immune reaction measurement reagent containing a mouse anti-human CRP polyclonal antibody and citric acid according to another example of the present invention or according to a comparative example.

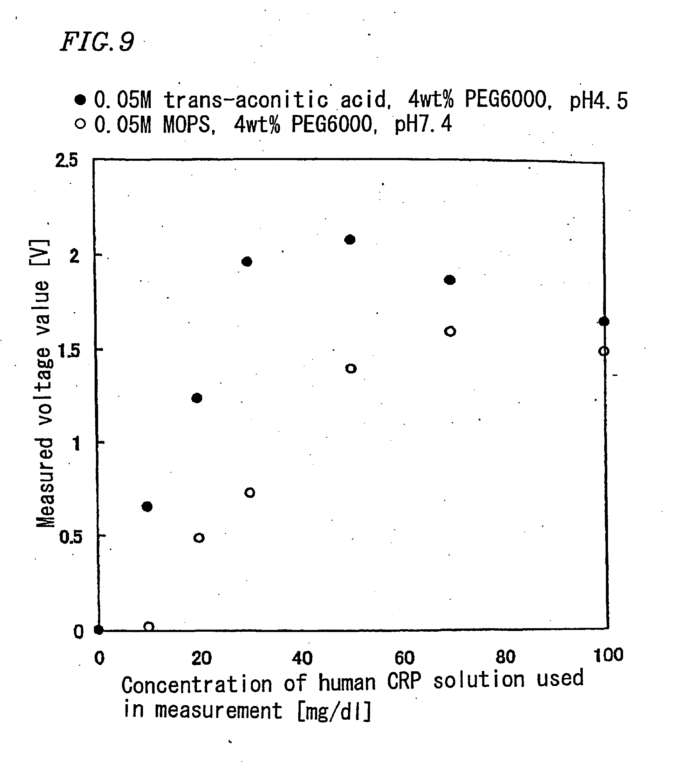

Figure **9** is a graph showing results of measurement of human CRP by immunological nephelometry using an immune reaction measurement method with an immune reaction measurement reagent containing a mouse anti-human CRP polyclonal antibody and trans-aconitic acid according to the example of the present invention or according to a comparative example.

### BEST MODE FOR CARRYING OUT THE INVENTION

The present invention relates to an immune reaction measurement method capable of easily increasing measurement values. The present invention also relates to an immune reaction measurement method capable of relaxing a zone phenomenon occurring in an antigen excess region.

The present inventors have found that by adding tricarboxylic acid or tricarboxylate into an antigen-antibody reaction system to maintain the pH of the reaction system as acidic, a measurement value of an immune reaction due to antigen-antibody binding can be improved and a zone phenomenon generated in an antigen excess region can be relaxed.

An immune reaction measurement method according to an embodiment of the present invention is a method for measuring an antigen or an antibody (subject substances) contained in a sample, characterized by comprising a step A of adding tricarboxylic acid or tricarboxylate and an antibody or an antigen capable of specifically binding to the subject substance (specifically binding substances) into the sample, and a step B of detecting an antigen-antibody complex generated by an antigen-antibody reaction between the subject substance and the specifically binding substance in the reaction system comprising the sample, the specifically binding substance and the tricarboxylic acid or the tricarboxylate composed by step A, and in that the pH of the reaction system when the antigen-antibody reaction is carried out is 4.5. Both tricarboxylic acid and tricarboxylate may be contained in the reaction system. Further, it is preferably that the tricarboxylic acid or tricarboxylate contained in the reaction system confer buffering capability thereto so that the pH of the reaction system is 4.5. In this case, no other buffer is required for setting the pH of the reaction system to be 4.5, and an increase in the measurement value of the immune reaction can be effectively achieved, efficiently leading to relaxation of a zone phenomenon occurring in an antigen excess region. In order to obtain buffering capability conferred by tricarboxylic acid or tricarboxylate contained in a reaction system, the tricarboxylic acid or tricarboxylate concentration is preferably at least 0.01 M but no more than 0.2M. An additional buffer may be further added to the reaction system.

As the buffer used in the immune reaction measurement method of the present invention, known buffers in tne art can be used, for example, including phosphate buffer (e.g., monosodium dihydrogen phosphate, disodium hydrogen phosphate, etc.), sodium acetate, sodium cacodylate, 2-(N-morpholino)ethanesulfonic acid, succinic acid, and the like. In this case, the amount of buffer contained may be regulated according to the type of the buffer, the amount of a sample (specimen) containing a subject substance, a method for supplying an antibody or an antigen relative to an antigen or an antibody (subject substances) in a reaction system, and the like, so as to achieve the effect of the present invention.

In the immune reaction measurement method of the present invention, the pH of the reaction system is set to be 4.5. When the pH is 4.5, an increase in measurement values of an immune reaction due to tricarboxylic acid or tricarboxylate is large, leading to enhancement of relaxation of a zone phenomenon occurring in an antigen excess region.

In the immune reaction measurement method of the present invention, the tricarboxylic acid or tricarboxylate concentration of the reaction system is no more than 0.2 M. In this case, an increase in measurement values of an immune reaction due to tricarboxylic acid or tricarboxylate is large, leading to enhancement of relaxation of a zone phenomenon occurring in an antigen excess region. When the tricarboxylic acid or tricarboxylate concentration of the reaction system is no more than 0.1 M, these effects are particularly preferably higher.

Examples of the tricarboxylic acid or tricarboxylate used in the immune reaction measurement method of the present invention, include citric acid, isocitric acid, aconiticacid, and salts thereof, which are commercially available in the form of citric anhydride, citric acid monohydrate, trisodium citrate, trisodium citrate dihydrate, potassium dihydrogen citrate, tripotassium citrate monohydrate, triammonium citrate, diammonium hydrogen citrate, calcium citrate tetrahydrate, magnesium citrate nonahydrate, trilithium citrate tetrahydrate, copper (II) citrate 2.5-hydrate, trisodium DL-isocitrate, trans-aconitic acid, and cis-aconitic anhydride, which can be used alone or in combination. Among them, preferable tricarboxylic acids or tricarboxylates are citric acid, citrate, aconitic acid, or aconitate, since they are relatively inexpensive, preservable at room temperature, stable, and easy to handle. More preferably, the aconitic acid is trans-aconitic acid.

Any other components known in the art may be added to the reaction system of the immune reaction measurement method of the present invention of the present invention in an amount which leads to the effect of the present invention, depending on the application. For example, when the present invention is applied to a homogeneous immune reaction measurement method, such as nephelometry, turbidimetry, slide agglutination, or the like, polyethylene glycol may be added to the reaction system of the immune reaction measurement method of the present invention of the present invention. Such a component is preferably contained in the reaction system in the immune reaction measurement method of the present invention at a concentration of 2 to 6% by weight, since such a content leads to less non-specific agglutination and a large improvement in measurement sensitivity, and more preferably at a concentration of 4% by weight concentration.

In order to reduce non-specific cloudiness due to autoagglutination of an antigen or an antibody, a surfactant, suchasTween20, octylglucoside, sodiumlaurylsulfate (SDS), sucrose monolaurate, CHAPS, or the like, may be added to the reaction system in the immune reaction measurement method of the present invention of the present invention. The surfactant is preferably contained at a concentration of no more than 0.3% relative to the reaction system in the immune reaction measurement method of the present invention, since such a content leads to less inhibition of an antigen-antibody reaction, and more preferably no more than 0.1%.

The immune reaction measurement method of the present invention is preferably applied to a homogeneous measurement system including, but being limited to, nephelometry, turbidimetry, and slide agglutination, which have a zone phenomenon generated in an antigen excess region. In this case, an increased effect can be preferably expected. In particular, when the present invention is applied to nephelometry and turbidimetry which are widely used for measurement using an automated measurement apparatus, steps required for determining a zone phenomenon generated in an antigen excess region can be preferably removed or simplified.

In the immune reaction measurement method of the present invention, the antigen-antibody complex is preferably an agglutination complex. In step B, the agglutination complex is preferably detected by measuring optical variations attributed to the agglutination complex. More preferably, the optical variation is a change in light scattering intensity or transmitted light amount.

An antigen or an antibody as a subject substance in the immune reaction measurement method of the present invention is not particularly limited and includes any substance capable of being measured generally using an antigen-antibody reaction, such as, for example, proteins, nucleic acids, lipids, bacteria, viruses, haptens, and the like. Among them, proteins, which are the major subject substances to be measured inclinical tests using an antigen-antibody reaction, are preferable. Examples of the proteins include hormones (e.g., LH (luteinizing hormone), FSH (follicle-stimulating hormone), hCG (human-chorionic gonadotropin), etc.), various immunoglobulin classes or their subclasses, complement components, various markers for infectious diseases, human C-reactive proteins (hereinafter abbreviated as human CRP), albumins, rheumatoid factors, blood group antigens, and the like. Among them, the subject substance is preferably human albumin or CRP.

Tricarboxylic acid and tricarboxylate have chelation ability, i.e., the property of efficiently sequestering bivalent and trivalent metal ions, such as Ca²⁺, Fe³⁺, or the like, from a reaction system. Therefore, when an antigen has a metal ion in its molecular structure, it is preferable that an antibody, which specifically binds to the antigen, still specifically binds to the antigen when the metal ion is released from the antigen. In this case, even when an antigen has a metal ion in its molecular structure and the molecular structure is changed by release of the metal ion, the antigen can be measured.

When an antigen has a metal ion in its molecular structure and the molecular structure is changed by release of the metal ion, the same metal ion as that contained in the antigen may be added to a reaction system so that the metal ion is present in the reaction system when an antigen-antibody reaction occurs.

The amount of a metal ion added may be determined based on the chelation ability or concentration of tricarboxylic acid or tricarboxylate used, the metal ion holding ability of an antigen, or the like.

An example of an antigen having a metal ion in its molecular structure is CRP, whose structure is changed depending on the presence or absence of Ca²⁺ in the structure. When the antigen in the immune reaction measurement method of the present invention is human CRP, goat anti-human CRP polyclonal antibodies including an antibody incapable of binding human CRP holding no Ca²⁺, and citric acid is used as tricarboxylic acid, 0.02 M Ca²⁺ is preferably added to a reaction system relative to 0.02 M citric acid.

When an antigen has a plurality of binding sites for a single antibody, the antibody is preferably a monoclonal antibody capable of binding to the plurality of binding sites of the antigen. A monoclonal antibody is produced by a hybridoma cell line. A hybridoma cell line is established by isolating and culturing a single cell from fusion cells having both an ability to produce an antibody and ability to proliferate, which is obtained by cell fusion of a B cell capable of producing an antibody and a myeloma cell. Antibodies produced by the hybridoma cell line all have the same properties. A hybridoma cell line has a strong proliferating ability and can be cryopreserved. If a hybridoma cell line is appropriately controlled, the cell line will not be exhausted. By culturing a hybridoma cell line in culture medium or an abdominal cavity and purifying it, antibodies having the same properties can be obtained perpetually. On the other hand, polyclonal antibodies can be obtained by injecting an antigen into an animal to allow a number of antibodies capable of binding to the antigen to appear in blood, and collecting and purifying the entirety or part of the blood. Therefore, the properties of the polyclonal antibody are dependent on the individual difference, feeding environment, conditions, or the like of the animal. Therefore, it is difficult to continue to obtain antibodies having the same properties. Thus, when a monoclonal antibody is used, it is possible to consistently use an antibody having the same property and stably supply the antibody as a reagent. As a result, it is possible to obtain stable results of immune reaction measurement using an immune reaction measurement method.

An antibody used in the immune reaction measurement method of the present invention is not particularly limited and may include an antibody of any type of IgG, IgM, IgE, IgA, and IgD as long as it specifically binds to an antigen. Among them, an IgG antibody is preferable, since the IgG antibody has less non-specific reactivity and is relatively often commercially available, i.e., easily obtainable. The type of an animal from which an antibody is derived is not limited and includes rabbit, goat, and mouse. Antibodies derived therefrom are relatively easily available and are generally used.

The immune reaction measurement method of the present invention can be typically performed as follows. Tricarboxylic acid or tricarboxylate is added to a buffer solution containing buffer in order to maintain an acidic pH of 4.5 of the reaction system. The tricarboxylic acid or tricarboxylate concentration is no more than 0.2.M in an antigen-antibody reaction, and particularly preferably no more than 0.1 M. The tricarboxylic acid or tricarboxylate may also serve as a buffer. One of a solution or a sample (specimen) containing an antibody or an antigen for an antigen or an antibody (subject substances) is mixed with the buffer solution, and the other is mixed with the resultant buffer solution to constitute the reaction system. An immune reaction generated in the reaction system is measured.

A method of adding tricarboxylic acid or tricarboxylate, a method of adding a buffer to a reaction system so as to keep the pH thereof at 4.5, and a method of adjusting the pH of a reaction system, are not limited to the above-described methods. For example, tricarboxylic acid or tricarboxylate and a buffer may be caused to be present in a solution containing an antibody or an antigen for an antigen or an antibody (subject substances) in such a manner as to satisfy the above-described requirements.

An immune reaction measurement reagent not forming part of the present invention may be typically prepared as follows.

When an antibody or an antigen for an antigen or an antibody (subject substances) and tricarboxylic acid or tricarboxylate are separately prepared, the procedure is performed as follows. A solution containing an antibody or an antigen for an antigen or an antibody (subject substances) can have any composition as long as the effect of the tricarboxylic acid or tricarboxylate can be obtained. The pH of a solution containing tricarboxylic acid or tricarboxylate is 4.5. The buffer and tricarboxylic acid or tricarboxylate are dissolved in pure water while adjusting the concentrations thereof so that the tricarboxylic acid or tricarboxylate concentration in an antigen-antibody reaction is no more than 0.8 M, and particularly preferably no more than 0.1 M. If the above-described requirements are satisfied, the buffer and tricarboxylic acid or tricarboxylate may be present in separate solutions. The tricarboxylic acid or tricarboxylate may also serve as the buffer.

Tricarboxylic acid or tricarboxylate may be present in a solution containing an antibody or an antigen for an antigen or an antibody (subject substances). In this case, tricarboxylic acid or tricarboxylate may be caused to be contained in a solution containing an antibody or an antigen for an antigen or an antibody (subject substances) by subjecting the solution to dialysis or gel filtration, using a solution containing tricarboxylic acid or tricarboxylate prepared in such a manner as to satisfy the above-described requirements, so as to exchange low molecular weight components.

As described above, according to the immune reaction measurement method of the present invention, tricarboxylic acid or tricarboxylate is caused to be present in an immune reaction system, and the pH of the reaction system is set to be 4.5, thereby increasing measurement values of an immune reaction due to antigen-antibody binding, and making it possible to relax a zone phenomenon generated in an antigen excess region. In conventional methods in which a water-soluble polymer is added, water-soluble polymer has to be added to a high concentration or water-soluble polymer having a high molecular weight has to be added, in order to increase measurement values in measurement of an antigen-antibody reaction to perform stable measurement while keeping a satisfactory S/N ratio. Unfortunately, in this case, the viscosity of the solution is increased, thereby making it difficult to handle the solution for analysis and manipulation. On the other hand, tricarboxylic acid or tricarboxylate used in the present invention is a low molecular weight substance, thereby avoiding an increase in the viscosity of the solution and making it easy to handle the solution for analysis and manipulation.

Further, a zone phenomenon generated in an antigen excess region can be relaxed, thereby reducing a decrease in a measurement value when the concentration of a subject substance is high. Therefore, a region in which a measurement value is high and a sample is determined to be positive can be broadened, thereby enlarging the range of measurable concentrations.

### Examples

Hereinafter, the present invention will be described by way of examples. The present invention is not limited only to these examples.

### (Example 1)

Hereinafter, a method for preparing a reagent will be described, where human albumin was used as a subject substance. In this example, a method for preparing a reagent comprising an antibody solution and a buffer solution containing tricarboxylic acid or tricarboxylate, which can be used for measurement employing slide agglutination, turbidimetry, or nephelometry, will be described.

A buffer solution and the like described below was prepared using pure water filtered with Milli-Q SP TOC (manufactured by Millipore). Any reagents, such as salts, buffers, and the like, which are not particularly described, were obtained from Wako Pure Chemical Industries. Polyethylene glycol 6000 and trans-aconitic acid were extra pure reagents , and other reagents were guaranteed reagents.

Initially, an antibody solution was prepared. Rabbit anti-human albumin polyclonal antibody was purified by protein A column chromatography from antiserum collected from rabbits immunized with human albumin. Protein A fixed gel was obtained from Amersham Pharmacia. Equilibrated buffer solution used for purification contained 1.5 M glycine and 3.0 M NaCl and had a pH of 8.9. Elution buffer solution contained 0.1 M citric acid and had a pH of 4.0. Purification was conducted as follows. An equilibrated buffer solution having a volume 5 times greater than the volume of the gel loaded in the column was passed through the column to equilibrate the column, after which an antiserum containing antibodies having an amount corresponding to 10 to 20% of the overall binding amount of the column was double diluted with an equilibrated buffer solution. The diluent was passed through the column to allow the antibodies in the serum to bind to protein A. Thereafter, the equilibrated buffer solution was continued to be passed until serum components incapable of being adsorbed to protein A did not come out of the column, whereby the column was washed. Thereafter, the elution buffer solution was passed through the column to elute antibodies binding to protein A. The eluted antibody fraction was placed in dialysis tubing with a molecular weight cutoff of 10,000. Dialysis was performed several times using an about 100-fold volume of buffer solution containing 0.05 M 3-(N-morpholino)propanesulfonic acid (manufactured by Dojin, hereinafter abbreviated as MOPS), 0.15 M NaCl, 0.04 wt% NaN₃ and having a pH of 7.4, to exchange components in the buffer solution. Thereafter, the antibody concentration was estimated based on measurement of absorbance at 280 nm. The antibody concentration was adjusted with the same buffer solution as used in the dialysis to 3.0 mg/ml. The resultant solution was regarded as an antibody solution. The antibody concentration was not so limited. The prepared antibody solution can be preserved at room temperature, however, is preferably preserved at low temperature to prevent denaturation of antibodies, and more preferably at 4°C.

A buffer solution containing tricarboxylic acid or tricarboxylate was prepared as follows. Citric acid and trans-aconitic acid were used as tricarboxylic acid or tricarboxylate to prepare two buffer solutions.

The buffer solution containing citric acid was prepared as follows. Citric acid monohydrate was weighed to an amount corresponding to a final concentration of 0.05 M. Polyethylene glycol 6000 was weighed to an amount corresponding to a final concentration of 4 wt%. The citric acid monohydrate and the polyethylene glycol 6000 were dissolved in pure water having a volume which was about 90% of a target preparation volume. An aqueous NaOH solution was added to the resultant solution to adjust the pH to be 4.5, and the resultant solution was adjusted with pure water to a target volume. The prepared buffer solution was preserved at room temperature.

The buffer solution using trans-aconitic acid was prepared as follows. Trans-aconitic acid was weighed to an amount corresponding to a final concentration of 0.05 M. Polyethylene glycol 6000 was weighed to an amount corresponding to a final concentration of 4 wt%. The trans-aconitic acid and the polyethylene glycol 6000 were dissolved in pure water having a volume which was about 90% of a target preparation volume. An aqueous NaOH solution was added to the resultant solution to adjust the pH to be 4.5, and the resultant solution was adjusted with pure water to a target volume. The prepared buffer solution was preserved at room temperature.

At least one of the thus-prepared buffer solutions containing tricarboxylic acid or tricarboxylate was combined with the antibody solution to prepare an immune reaction measurement reagent.

### (Example 2)

Next, a method of preparing a reagent for human CRP as a subject substance will be described below. Human CRP is a substance composed of 5 subunits having the same structure and therefore having a plurality of binding sites for a single antibody. Therefore, a single anti-CRP monoclonal antibody can be used to prepare a reagent for use in homogeneous immune reaction measurement. More than one monoclonal antibody may be used.

In this example, a reagent comprising two antibody solutions, i.e., a polyclonal antibody solution and a monoclonal antibody solution, and a buffer solution containing tricarboxylic acid or tricarboxylate was prepared.

Firstly, a method of preparing a reagent using a polyclonal antibody solution will be described. The antibody solution was prepared as follows. Goat anti-human CRP polyclonal antibody was purified from antiserum collected from a goat immunized with human CRP using protein G column chromatography. Protein G fixed gel loaded in the column was obtained from Amersham Pharmacia. Equilibrated buffer solution used for purification contained 0.02 M Na₂HPO₄-NaH₂PO₄ and had a pH of 7.0. Elution buffer solution contained 0.1 M glycine and had a pH of 2.7. Purification by column chromatography and buffer solution exchange by dialysis were performed in a manner similar to that in Example 1. Thereafter, the antibody concentration was estimated based on measurement of absorbance at 280 nm. The antibody concentration was adjusted with the same buffer solution as used for dialysis to 1.0 mg/ml, resulting in an antibody solution.

Human CRP changes its structure depending on the presence or absence of Ca²⁺ bound. Therefore, when antibodies constituting a reagent include an antibody incapable of binding to human CRP without Ca²⁺, chelation due to tricarboxylic acid or tricarboxylate leads to an increase in human CRP without Ca²⁺, thereby potentially reducing the rate of an antigen-antibody reaction. The antibody solution prepared above contained polyclonal antibodies including an antibody incapable of binding to human CRP without Ca²⁺. Therefore, Ca²⁺ was added to a buffer solution described below containing tricarboxylic acid or tricarboxylate in order to maintain the structure of human CRP.

A buffer solution containing tricarboxylic acid or tricarboxylate was prepared as follows. As tricarboxylic acid or tricarboxylate, citric acid was used.

Citric acid monohydrate was weighed to an amount corresponding to a final concentration of 0.02 M. CaCl₂ was weighed to an amount corresponding to a final concentration of 0.02 M. Polyethylene glycol 6000 was weighed to an amount corresponding to a final concentration of 4 wt%. The citric acid monohydrate, CaCl₂ and polyethylene glycol 6000 were dissolved in pure water having a volume which was about 90% of a target preparation volume. An aqueous NaOH solution was added to the resultant solution to adjust the pH to be 4.5, and the resultant solution was adjusted with pure water to a target volume. The prepared buffer solution was preserved at room temperature.

Next, a reagent using a monoclonal antibody solution will be described. As a monoclonal antibody, an antibody was used which does not lose the ability to bind to human CRP even when a chelating agent, such as 0.02 M ethylenediamine tetraacetic acid, is added to a reaction system, i.e., which can specifically bind to human CRP without bound Ca²⁺ when Ca²⁺ is released from the human CRP.

An antibody solution was prepared as follows. A mouse anti-human CRP monoclonal antibody used in this example was obtained by injecting a hybridoma cell producing the antibody (National Institute of Bioscience and Human-Technology Agency of Industrial Science and Technology, deposit number: FERM BP-6620) into a mouse abdominal cavity, followed by proliferation. The antibody was purified from the resultant ascites fluid by column chromatography as described in Example 1.

The ascites fluid was obtained as follows. The ascites fluid was produced using retired female BALB/c mice. A suspension of the hybridoma cells, which was injected into the abdominal cavity, was prepared by proliferating hybridoma cells in RPMI1640 medium (manufactured by SIGMA) mixed with 5 to 15 % by volume fetal calf serum, followed by centrifugal washing with RPMI1640 medium, and resuspending in RPMI1640 medium to a concentration of 1×10⁶ to 10⁷ cells/ml. 0.5 to 1 ml of pristane was injected into the abdominal cavity of a mouse. After about 7 days, 0.5 to 1 ml of the above-described suspension was injected into the mouse. When production of ascites fluid was observed, the ascites fluid was collected from the mouse.

An antibody sample after purification by column chromatography was placed in dialysis tubing with a molecular weight cutoff of 10,000, followed by dialysis several times using an about 100-fold volume of a PBS buffer solution containing 0.04 wt% NaN₃ (8 g/l NaCl, 0.2 g/l KCl, 1.15 g/l Na₂HPO₄ ·12H₂O, 0.2 g/l KH₂PO₄, pH7.4) to exchange components of the buffer solution. Thereafter, the antibody concentration was estimated based on measurement of absorbance at 280 nm. The antibody concentration was adjusted with the same buffer solution as used for dialysis to 1.0 mg/ml, resulting in an antibody solution.

A buffer solution containing tricarboxylic acid or tricarboxylate was prepared as follows. Citric acid and trans-aconitic acid were used as tricarboxylic acid or tricarboxylate to prepare two buffer solutions. In this example, the antibodies were not affected by a change in the structure of human CRP due to the presence or absence of Ca²⁺ held in the human CRP. Therefore, Ca²⁺ was not added to the buffer solution.

The buffer solution containing citric acid was prepared as follows. Citric acid monohydrate was weighed to an amount corresponding to a final concentration of 0.05 M. Polyethylene glycol 6000 was weighed to an amount corresponding to a final concentration of 4 wt%. The citric acid monohydrate and the polyethylene glycol 6000 were dissolved in pure water having a volume which was about 90% of a target preparation volume. An aqueous NaOH solution was added to the resultant solution to adjust the pH to be 4.5, and the resultant solution was adjusted with pure water to a target volume. The prepared buffer solution was preserved at room temperature.

The buffer solution using trans-aconitic acid was prepared as follows. Trans-aconitic acid was weighed to an amount corresponding to a final concentration of 0.05 M. Polyethylene glycol 6000 was weighed to an amount corresponding to a final concentration of 4 wt%. The trans-aconitic acid and the polyethylene glycol 6000 were dissolved in pure water having a volume which was about 90% of a target preparation volume. An aqueous NaOH solution was added to the resultant solution to adjust the pH to be 4. 5, and the resultant solution was adjusted with pure water to a target volume. The prepared buffer solution was preserved at room temperature.

The concentration of the antibody solution prepared above was not so limited. The prepared antibody solution can be preserved at room temperature, however, is preferably preserved at low temperature for prevention of denaturation of antibodies, and more preferably at 4°C.

At least one of the thus-prepared buffer solutions containing tricarboxylic acid or tricarboxylate was combined with the antibody solution to prepare an immune reaction measurement reagent.

A method of using the reagents prepared in Examples 1 and 2 is to mix a sample (specimen) containing an antigen, an antibody solution, and a buffer solution containing tricarboxylic acid or tricarboxylate so as to prepare a reaction system. Any mixing method may be used. The mixture ratio can be determined depending on the measurement range of the required antigen concentration. By measuring an immune reaction between the antigen and the antibody generated in the reaction system prepared by the mixture, it is possible to determine the antigen concentration in the specimen.

By the mixing, additives, such as a buffer, tricarboxylic acid or tricarboxylate, polyethylene glycol 6000, and the like, were diluted to concentrations lower than their initial concentrations. If the difference between the diluted concentration and the initial concentration is within about 10%, the results of the measurement are not much different from the measurement results expected from the initial concentrations, i.e. , are not much affected. In order to avoid variations in the concentration due to dilution, each substance in a reagent can be prepared in such a manner as to take into consideration dilution due to mixing and allow the concentration of each substance to be a target concentration.

Note that an antibody may be fixed to a small particulate carrier, such as a latex, a gold colloid, a magnetic particulate, or alternatively, an antibody may be labeled with an enzyme, a pigment, a fluorescent substance, a luminescent substance, or the like, although not described in Examples 1 and 2.

The buffer component and pH of the antibody solution are not limited to the above-described composition and pH. For example, when one-component reagent is prepared, dialysis may be performed using an acidic buffer solution containing tricarboxylic acid or tricarboxylate in order to allow the antibody solution to contain tricarboxylic acid or tricarboxylate and to keep the pH of a reaction system acidic.

In Examples 1 and 2, NaOH was used for pH adjustment. Alternatively, hydroxide, such as KOH, LiOH, NH₄OH, Ca(OH)₂, Mg(OH)₂ or the like, may be used.

In Examples 1 and 2, as tricarboxylic acid or tricarboxylate contained in a buffer solution, citric acid monohydrate and trans-aconitic acid were used. Other tricarboxylic acids or tricarboxylates may be used, including, for example, isocitric acid, citric anhydride, trisodium citrate, trisodium citrate dihydrate, potassium dihydrogen citrate, tripotassium citrate monohydrate, triammonium citrate, diammonium hydrogen citrate, calcium citrate tetrahydrate, magnesium citrate nonahydrate, trilithium citrate tetrahydrate, copper (II) citrate 2.5 hydrate, trisodium DL-isocitrate, and cis-aconitic anhydride, which may be used alone or in combination. In the combination use, the pH may be adjusted as follows. If the pH is more alkaline than a target pH in the dissolution in pure water, HCl or the like is used. If the pH is more acidic, the above-described hydroxide or the like may be used. Alternatively, the mixture ratio of tricarboxylic acid or tricarboxylate may be used for pH adjustment.

In Examples 1 and 2, a buffer solution containing tricarboxylic acid or tricarboxylate was conferred buffering capability mainly by the tricarboxylic acid or tricarboxylate. The concentration of tricarboxylic acid or tricarboxylate to be added to a reagent is not particularly limited. Alternatively, another buffer may be used to confer main buffering capability to a reagent, or to confer buffering capability in cooperation with tricarboxylic acid.

### (Example 3)

In this example, the effect of an acidic reaction system containing tricarboxylic acid or tricarboxylate on an antigen-antibody reaction was shown, compared with a neutral reaction system generally used in an immune reaction measurement method. Comparison was performed by measuring human albumin using immunological nephelometry. A reagent as prepared in Example 1 was used for preparing an acidic reaction system containing tricarboxylic acid or tricarboxylate.

Hereinafter, as a buffer solution containing tricarboxylic acid or tricarboxylate, a buffer solution containing citric acid or its salt as a main buffer and having a similar composition as prepared in Example 1 is referred to as a citrate buffer solution, and a buffer solution containing trans-aconitic acid or its salt as a main buffer and having a similar composition as prepared in Example 1 is referred to as an aconitate buffer solution.

As a comparative example, MOPS was used to prepare a buffer solution for constituting a neutral reaction system, and the concentration and pH of the buffer solution were set to be commonly used values, i.e., 0.05 M MOPS, 4 wt% polyethylene glycol 6000, and pH 7.4. Hereinafter, it is referred to as a MOPS buffer solution. The same antibody solution was shared by the citrate buffer solution and the aconitate buffer solution.

Human albumin (manufactured by Wako Pure Chemical Industries) as an antigen was dissolved in a buffer solution (0.05 M MOPS, pH 7.4) to a concentration of 0, 5, 10, 30, 50, or 100 mg/dl. An antibody and an antigen solution (sample) were preserved at 4°C before use, and each buffer solution was preserved at room temperature.

A self-made apparatus was used for measurement, which was constructed as followed. A semiconductor laser pointer having a wavelength of 680 nm modulated at 270 Hz and an output power of 15 mW (manufactured by Kikoh Giken, model number: MLXS-D-12-680-35) was used as a light source. A visible and infrared light precision measurement silicon photodiode (manufactured by Hamamatsu Photonics, model number: S2387-66R) was used as a detector. A cell was constructed by attaching optical glass plates having a thickness of 0.1 cm together, which was in the shape of a square prism having a volume of about 200 µl. The cell was disposed 0.5 cm away from the light source, one side of the cell being perpendicular to the light source. The detector was disposed 5.5 cm away from the cell and at an angle of 90° with respect to the light source. A light shielding tube was placed between the detector and the cell so as to prevent stray light from entering the detector. A current signal depending on the amount of light detected by the detector was amplified by a current-voltage conversion circuit (10⁶ V/A) and an amplifier (operational amplifier) to a 100-fold voltage signal. Thereafter, the voltage signal was passed through a lock-in amplifier (manufactured by NF Corporation, model number: 5610B) to perform phase-sensitive detection and then input into a computer by GPIB control.

For each buffer solution, human albumin having each concentration was measured as follows. The mixture ratio of a reaction system was 178 µl of a buffer solution, 9 µl of a human albumin solution, and 7 µl of an antibody solution. The final concentrations of an antibody and human albumin in a reaction system were about 0.11 mg/ml and the concentration of the human albumin solution used in measurement multiplied by 0.046, respectively.

Firstly, the buffer solution and the human albumin solution having the above-described volumes were added to the cell, followed by mixing by stirring. Thereafter, the antibody solution having the above-described volume was added to the cell, followed by mixing by stirring, causing an antigen-antibody reaction. Measurement of scattered light was started 10 seconds before the addition of the antibody solution, and was continued every 0.5 seconds for 300 seconds. The measurement values were obtained as voltage values. An influence of contamination of the cell on measurement was removed by correcting the measurement values based on measurement which had been conducted, where pure water was placed in the cell, before measuring each reaction. The measurement values obtained at respective times were averaged over 200 to 300 seconds. The resultant average value was regarded as a measurement value for a human albumin solution having each concentration. When occurrence of autoagglutination of an antigen was determined based on the measurement values obtained for 10 seconds before the addition of the antibody solution, the average value of the measurement values was subtracted from the measurement values for a human albumin solution having each concentration. The measurement was performed at room temperature (about 20°C).

After measurement, an influence of mixture of each buffer solution, the antibody solution, and the human albumin solution having each concentration on the pH of a reaction system was observed by measuring the pH of the mixture using apHmeter (manufactured by Shindengen Electric Manufacturing, trade name: pHBOY-P2).

The results were that the pH of the mixture of each buffer solution, the antibody solution, and the human albumin solution having each concentration, which was used for each measurement, was the same as the pH of the original buffer solution.

Figure **1** shows plots representing results of measurement of the human albumin solution having each concentration up to 100 mg/dl with respect to each buffer solution. The vertical axis represents a voltage value, while the horizontal axis represents the concentration of the human albumin solution used for measurement. Figure 1 indicates that the higher the measured voltage value, the larger the amount of scattered light entering the detector. The larger amount of scattered light indicates the higher cloudiness of a reaction system and the larger amount of an antigen-antibody complex generated by an antigen-antibody reaction. The plotted value was obtained by subtracting a measurement value (0 mg/dl), obtained when a buffer solution did not contain human albumin, from a measurement value of the human albumin solution having each concentration with respect to the same buffer solution.

As shown in Figure **1**, measurement values are higher when a citrate buffer solution and an aconitate buffer solution were used for measurement of an antigen-antibody reaction (indicated by filled circles and unfilled circles, respectively, in Figure **1**) than when a comparative example, MOPS buffer solution, was used (indicated by filled triangles in Figure **1**). When the MOPS buffer solution was used, the measurement value was much reduced from the peak around 30 mg/dl due to a zone phenomenon generated in an antigen excess region. In contrast, when the citrate buffer solution was used, the peak was present around 30 mg/dl similar to the MOPS buffer solution, a reduction in a measurement value due to a zone phenomenon generated in an antigen excess region was suppressed. When the aconitate buffer solution was used, the concentration at the peak was observed to be slightly changed, and a reduction in a measurement value due to a zone phenomenon generated in an antigen excess region was further suppressed.

According to the above-described results, it could be confirmed that the immune reaction measurement method of the present invention can be used to increase the measurement values of an antigen-antibody reaction. Therefore, it could also be confirmed that a zone phenomenon generated in an antigen excess region can be relaxed.

In a clinical test, a trace amount of albumin excreted in urine is measured as a marker for early diagnosis of diabetic nephropathy. The range of 0.1 to 20 mg/dl is used as a quantification region in a number of measurement methods and reagents (see Shin-Tonyobyosei-Jinsho Hasashoyobo-to-Shintenbosh1 [New Diabetic Nephropathy, for Prevention of Onset and Progression], Yukio Shigeta, Yoshizo Umitsu ed., p. 131 (1992)). In the case of a conventional neutral buffer solution, which is used in measurement employing immunological nephelometry and which constitutes a reagent used such measurement, a zone phenomenon generated in an antigen excess region has to be removed, based on the fact that a homogeneous immune reaction is a kind of equilibrium reaction, by increasing the antibody concentration or decreasing the antibody concentration by dilution. According to the immune reaction measurement method of the present invention, a method for measuring human albumin can be provided, in which the antibody concentration can be lower and dilution of an antigen is not required, and a zone phenomenon generated in an antigen excess region can be removed. For example, according to the measurement results in this example, by providing a determination region where a measurement value of at least 20 mg/dl is positive, human albumin having a concentration range wider than that which can be measured using a conventional neutral buffer solution system, can be measured without considering an influence of a zone phenomenon generated in an antigen excess region.

### (Example 4)

Next, citric acid and trans-aconitic acid were used as tricarboxylic acid or tricarboxylate to study the pH dependency of the effect thereof on an antigen-antibody reaction based on immunological nephelometry. The results will be described below. As a subject substance, human albumin was used. A human albumin solution was prepared in the same manner as described in Example 3. The concentration of the human albumin solution was 0, 5, 10, 30, 50, or 100 mg/dl. An antibody solution as described in Example 1 was used.

In order to study the pH dependency of citric acid, a solution containing 0.05 M citric acid and 4 wt% polyethylene glycol 6000 and a solution containing 0.05 M trisodium citrate and 4 wt% polyethylene glycol 6000 were separately prepared and then were mixed together to obtain a citrate buffer solution having a pH of 3.5, 4.0, 4.5, 5.0, 5.5, or 6.0.

In order to study the pH dependency of aconitic acid, a solution containing 0.1 M trans-aconitic acid and 4 wt% polyethylene glycol 6000 was adjusted to obtain an aconitate buffer solution having a pH of 4.0, 4.5, 5.0, 5.5, or 6.0.

As a comparative example, a MOPS buffer solution was used. An apparatus and a measurement method were similar to those in Example 3. Measurement was performed at room temperature (about 20°C). After measurement, an influence of mixture of each buffer solution, the antibody solution, and the human albumin solution having each concentration on the pH of a reaction system was observed by measuring the pH of the mixture using a pH meter.

The results are shown in Figures **2** and **3**. The pH of the mixture of each buffer solution, the antibody solution, and the human albumin solution having each concentration, which was used for each measurement, was the same as the pH of the buffer solution.

Figure **2** shows plots representing results of measurement of the human albumin solution having each concentration up to 100 mg/dl with respect to a citrate buffer solution having each pH. The vertical axis represents a voltage value, while the horizontal axis represents the concentration of the human albumin solution used for measurement. Figure **3** shows plots representing results of measurement of the human albumin solution having each concentration up to 100 mg/dl with respect to an aconitate buffer solution. Similar to Figure **2**, the vertical axis represents a voltage value, while the horizontal axis represents the concentration of the human albumin solution used for measurement. Graphs in Figures **2** and **3** are assessed in the same manner as in Figure **1**. The plotted value was obtained by subtracting a measurement value (0 mg/dl), obtained when a buffer solution did not contain human albumin, from a measurement value of the human albumin solution having each concentration with respect to the same buffer solution.

Measurement using a citrate buffer solution in the pH range of 4.0 to 5.5 and an aconitate buffer solution in the pH range of 4.5 to 5.0 showed that quantification did not suffer much from troubles in a low concentration region and measurement values were increased as compared to the comparative example, MOPS buffer solution (indicated by x in Figures **2** and **3**). Further, a reduction in measurement values due to a zone phenomenon generated in an antigen excess region was relaxed. Such effects were largest at pH 4.5 for both of the buffers (indicated by filled triangles in Figure **2** and unfilled circles in Figure **3**).

In the case of a citrate buffer solution, when the pH was less than 4.0 (indicated by filled circles in Figure **2**) or 6.0 or more (indicated by unfilled squares in Figure **2**), no increase in measurement values was observed and the measurement values were lower than measurement values in the case of a MOPS buffer solution. Further, no relaxation of reduction in measurement values due to a zone phenomenon generated in an antigen excess region, was observed. On the other hand, in the case of an aconitate buffer solution, when the pH was 5.5 or more (indicated by unfilled triangles and filled squares in Figure **3**), no increase in measurement values was observed and the measurement values were substantially equal to or lower than the measurement values in the case of a MOPS buffer solution. Further, no relaxation of a reduction in measurement values due to a zone phenomenon generated in an antigen excess region, was observed.

Further, in the case of an aconitate buffer solution, when the pH was 4.0, non-specific cloudiness was increased and quantitative measurement suffered from troubles in a low concentration region. However, an increase in measurement values was observed and relaxation of a reduction in measurement values due to a zone phenomenon generated in an antigen excess region, was observed (indicated by filled circles in Figure **3**).

The above-described results are summarized below. It was found that considering an increase in measurement values, or thus relaxation of a reduction in measurement values due to a zone phenomenon generated in an antigen excess region, the pH of citric acid is preferably in the range of 4.0 to 6.0 and the pH of trans-aconitic acid is preferably in the range of 4.0 to 5.5. In particular, if quantification is also taken into consideration, both of the above-described effects were highest when pH was 4.5. It was found that pH 4.5 led to the highest effectiveness.

According to the above-described results, it was found that in an immune reaction measurement method using tricarboxylic acid or tricarboxylate, the pH of a reaction system is preferably set to be in the range of 4.0 to 6.0. It was also found that when the pH of a reaction system is set to be 4.5, the greatest effect can be obtained.

### (Example 5)

Next, citric acid and trans-aconitic acid were used as tricarboxylic acid or tricarboxylate to study the concentration dependency of the effect thereof on an antigen-antibody reaction based on immunological nephelometry. The results will be described below.

As a subject substance, human albumin was used. A human albumin solution was prepared in a manner as described in Example 3. The concentration of the human albumin solution prepared was 0, 5, 10, 30, 50, or 100 mg/dl in the case of an experiment on the concentration dependency of citric acid. The concentration of the human albumin solution prepared was 0, 10, 20, 30, 40, 60, 80, 100, or 200 mg/dl in the case of an experiment on the concentration dependency of trans-aconitic acid. An antibody solution as described in Example 1 was used.

In order to study the concentration dependency of citric acid, a citrate buffer solution (pH 4.5) containing 0.01, 0.02, 0.1, 0.2, or 0.3 M citric acid and 4 wt% polyethylene glycol 6000 was prepared.

In order to study the concentration dependency of aconitic acid, a trans-aconitate buffer solution (pH 4.5) containing 0.01, 0.02, 0.05, 0.1, 0.2, or 0.3 M trans-aconitic acid and 4 wt% polyethylene glycol 6000 was prepared.

As a comparative example, a MOPS buffer solution was used. The apparatus and measurement method were similar to those in Example 3, except that in the case of measurement using the trans-aconitate buffer solution, a current signal depending on the amount of light detected by a detector was amplified to an about 70-fold voltage signal. Measurement was performed at room temperature (about 20°C). After measurement, the influence of mixing of each buffer solution, the antibody solution, and the human albumin solution having each concentration on the pH of a reaction system was observed by measuring the pH of the mixture using a pH meter.

Results are described below. The pH of the mixture of each buffer solution, the antibody solution, and the human albumin solution having each concentration in each measurement was changed to pH 4.8 and 4.7 where 0.01 and 0.02 M citrate buffer solution and trans-aconitate buffer solution were used, respectively. In the case of the other buffer solutions, the pH of the mixture was the same as the buffer solution. According to the results in Example 4, an influence of the pH change was considered to be small and was ignored.

Figure **4** shows plots representing results of measurement of the citrate buffer solution having each concentration mixed with the human albumin solution having each concentration up to 100 mg/dl. Figure **5** shows plots representing results of measurement of the trans-aconitate buffer solution having each concentration mixed with the human albumin solution having each concentration up to 200 mg/dl.

In either figure, the vertical axis represents a voltage value, while the horizontal axis represents the concentration of the human albumin solution used for measurement. The graph in Figure **4** is assessed in the same manner as in Figure **1**. The plotted value was obtained by subtracting a measurement value (0 mg/dl), obtained when a buffer solution did not contain human albumin, from a measurement value of the human albumin solution having each concentration with respect to the same buffer solution.

Although mixing of the buffer solution, the antibody solution, and the human albumin solution led to a slight reduction in the citric acid and trans-aconitic acid concentrations, the magnitude of such a reduction was no more than 10%. Therefore, the mixing is not considered to have a significant influence on the result.

In the case of the citrate buffer solution, when the citric acid concentration was no more than 0.2 M (indicated by circles and triangles in Figure **4**), measurement values were higher than those in the case of the comparative example, a MOPS buffer solution (indicated by × in Figure **4**). An increase in measurement values could be confirmed. Thus, relaxation of a reduction in measurement values due to a zone phenomenon generated in an antigen excess region was also observed. The lower the concentration of the citrate buffer solution, the higher the above-described effects. When the citric acid concentration was 0.3 M (indicated by filled squares in Figure **4**), measurement values were lower than those in the case of a MOPS buffer solution. No increase in measurement values was observed. Also, no relaxation of reduction in measurement values due to a zone phenomenon generated in an antigen excess region was observed.

In the case of the trans-aconitate buffer solution, measurement values were higher for all of the concentrations of 0.01 to 0.3 M than those in the case of the comparative example, a MOPS buffer solution (indicated by × in Figure 5). That is, an increase in measurement values could be confirmed. Thus, relaxation of a reduction in measurement values due to a zone phenomenon generated in an antigen excess region was also observed. These effects were higher when the concentration was no more than 0.2 M, were particularly high when the concentration was no more than 0.1 M (indicated by circles and triangles in Figure 5), and were highest the concentration was 0.05 M (indicated by filled triangles in Figure **5**). When the concentration was in the range of 0.2 to 0.05 M (indicated by triangles and filled squares in Figure **5**), the lower the concentration of the trans-aconitate buffer solution, the higher the effects. A clear difference in the effects could not be confirmed when the concentration was no more than 0. 05 M (indicated by filled triangles and circles in Figure **5**).

The above-described results are summarized below. It was found that considering an increase in measurement values, or thus, relaxation of a reduction in measurement values due to a zone phenomenon generated in an antigen excess region, the citric acid concentration is preferably no more than 0.2 M and the trans-aconitic acid concentration is preferably no more than 0.3 M in order to obtain the effects higher than those in the case of a general neutral buffer solution. It was also found that the concentrations of both of the substances are more preferably no more than 0.1 M and, in this case, the effects can be improved.

According to the above-described results, it was found that in an immune reaction measurement method using tricarboxylic acid or tricarboxylate, the tricarboxylic acid or tricarboxylate concentration in a reaction system is preferably set to be no more than 0.3 M. Further, when the tricarboxylic acid or tricarboxylate concentration in a reaction system is set to be no more than 0.1 M, improved effects can be obtained.

Similarly, it was found that in an immune reaction measurement reagent using tricarboxylic acid or tricarboxylate, the tricarboxylic acid or tricarboxylate concentration in a reaction system is preferably set to be no more than 0.3 M. Further, when the tricarboxylic acid or tricarboxylate concentration in a reaction system is set to be no more than 0.1 M, improved effects can be obtained.

### (Example 6)

Next, the effect of tricarboxylic acid or tricarboxylate on an antigen-antibody reaction when used in the mixture with other buffer solutions, was confirmed using immunological nephelometry. Results are described below. Comparison was performed based on human albumin measurement. A human albumin solution was prepared in a manner as described in Example 3. The concentration of the human albumin solution was 0, 5, 10, 20, 30, 50, 70, 100, 200, 300, or 500 mg/dl. An antibody solution as described in Example 1 was used. As tricarboxylic acid or tricarboxylate, citric acid and trans-aconitic acid were used. Succinic acid was used together with the above-described buffer. A buffer solution (pH 4.5) containing 0.1 M succinic acid, 0.02 M citric acid, and 4 wt% polyethylene glycol 6000 and a buffer solution (pH 4.5) containing 0.1 M succinic acid, 0.02 M trans-aconitic acid, and 4 wt% polyethylene glycol 6000 were prepared. As a comparative example in which tricarboxylic acid or tricarboxylate was not present, a buffer solution (pH 4.5) containing 0.12 M succinic acid and 4 wt% polyethylene glycol 6000 was prepared.

For measurement, a fluorescence spectrophotometer (manufactured by Shimadzu Corporation, model number: RF-5300PC) was used. A constant-temperature cell holder (manufactured by Shimadzu Corporation, model number: 206-15440) was placed in a sample chamber of the spectrofluorometer. The constant-temperature cell holder was connected to a constant-temperature bath (manufactured by TAITEC, trade name: COOLNITBATHEL-15). Water maintained at 25°C was circulated through the bath so as to maintain constant temperature in measurement. Conditions for measurement using the spectrofluorometer were: excitation light and fluorescent light each had a wavelength of 670 nm, band width was 3 nm both at the fluorescence side and the excitation side, and sensitivity was set to be high.

Measurement was performed as follows. 2.87 ml of buffer solution and 0.1 ml of antibody solution were mixed together while stirring. To the mixture, 0.03 ml of a human albumin solution was added, followed by mixing while stirring. The final concentration of an antibody and human albumin in a reaction system was about 0.10 mg/ml and the concentration of the human albumin solution in measurement multiplied by 0.01, respectively. The mixture was transferred to a quartz cell for fluorescence analysis. The quartz cell was placed in the spectrofluorometer. A T-type thermocouple (obtained from RS Components, model number: 219-4696) was immersed in the cell. Time-course measurement was started 2 minutes after adding the human albumin, and was continued every 0.04 seconds for 300 seconds. Temperature in the cell during measurement was monitored with a digital multithermometer (manufactured by Advantest, model number: TR2114) connected to the T-type thermocouple. Any influence of contamination of the cell on measurement was removed by correcting measurement values based on measurement which had been conducted, where pure water was placed in the cell, before measuring each reaction. The measurement values obtained at respective times were averaged over 200 to 300 seconds. The resultant average value was regarded as a measurement value for a human albumin solution having each concentration. After measurement, the influence of mixing of each buffer solution, the antibody solution, and the human albumin solution having each concentration on the pH of a reaction system was observed by measuring the pH of the mixture using a pH meter.

Results are described below. The pH of the mixture of the buffer solution, the antibody solution, and the human albumin solution having each concentration in each measurement was the same as the pH of the buffer solution. The temperature in the cell during measurement, which was measured with the thermocouple, was held at 25.5±1°C.

Figure **6** shows plots representing results of measurement of the human albumin solution having each concentration up to 500 mg/dl with respect to each buffer solution. The vertical axis represents the intensity of scattered light, while the horizontal axis represents the concentration of the human albumin solution used for measurement. The plotted value was obtained by subtracting a measurement value (0 mg/dl), obtained when a buffer solution did not contain human albumin, from a measurement value of the human albumin solution having each concentration with respect to the same buffer solution.

As a result, the buffer solutions containing citric acid or trans-aconitic acid (indicated by circles in Figure **6**) increased measurement values as compared to the comparative example containing only succinic acid (indicated by filled triangles in Figure **6**). That is, the effect could be confirmed. Thus, relaxation of a reduction in measurement values due to a zone phenomenon generated in an antigen excess region was also observed.

According to the above-described results, even when tricarboxylic acid or tricarboxylate was used together with other buffers, the effect could be confirmed.

### (Example 7)

Next, the effect of the reagent using a goat anti-human CRP polyclonal antibody solution as prepared in Example 2 on human CRP measurement was compared with a neutral reaction system commonly used in an immune reaction measurement method. The results are described below. CRP solutions having each concentration used in measurement were prepared by diluting purified human CRP (manufactured by Chemicon International, Lot No. 21042246) with a buffer solution (pH 7.4) containing 0.05 M MOPS, 0.04 wt% and NaN₃. The concentration of the human CRP solution prepared was 0, 10, 20, 30, 50, 70, 100, or 200 mg/dl. An antibody solution contained a reagent using a polyclonal antibody solution as prepared in Example 2. As a comparative example in which tricarboxylic acid or tricarboxylate was not present, a MOPS buffer solution was used.

For measurement, an apparatus having the same arrangement as described in Example 3 and the same measurement conditions as described in Example 3 were used. The same measurement method and method for processing measured data as described in Example 3 were used, except that the antigen solution was different.

The final concentrations of antibody and human CRP in a reaction system were about 0.036 mg/ml and the concentration of the human CRP solution used in measurement multiplied by 0.046, respectively.

Results are shown below. Figure 7 shows plots representing results of measurement of the buffer solution mixed with the human CRP solution up to 200 mg/dl. The vertical axis represents a voltage value, while the horizontal axis represents the concentration of the human CRP solution used for measurement. The graph in Figure 7 is assessed in the same manner as in Figure **1**. The plotted value was obtained by subtracting a measurement value (0 mg/dl), obtained when a buffer solution did not contain human CRP, from a measurement value of the human CRP solution having each concentration with respect to the same buffer solution.

According to Figure **7**, as compared to when the comparative example, the MOPS buffer solution, was used to measure an antigen-antibody reaction (indicated by unfilled circles in Figure **7**), higher measurement values were clearly confirmed when measurement was performed using a reagent prepared from a buffer solution containing a goat anti-human CRP polyclonal antibody solution and 0.02 M CaCl₂ and citric acid as prepared in Example 2 (indicated by filled circles in Figure **7**).

### (Example 8)

Next, the effect of the reagent using a mouse anti-human CRP monoclonal antibody solution as prepared in Example 2 on human CRP measurement was compared with a neutral reaction system commonly used in an immune reaction measurement method. Results are described below.

Human CRP solutions having each concentration used in measurement were prepared in the same manner as described in Example 7. The concentration of the human CRP solution prepared was 0, 10, 20, 30, 50, 70, or 100 mg/dl. Theantibody solution contained a reagent using a monoclonal antibody solution as prepared in Example 2. As a comparative example in which tricarboxylic acid or tricarboxylate was not present, a MOPS buffer solution was used.

For measurement using a reagent containing citric acid as tricarboxylic acid or tricarboxylate, an apparatus having the same arrangement as described in Example 6 and the same measurement conditions as described in Example 6 were used. The same measurement method and method for processing measured data as described in Example 6 were used, except that the antigen solution was different.

The final concentrations of an antibody and human CRP in a reaction system were about 0.033 mg/ml and the concentration of the human CRP solution used in measurement multiplied by 0.010, respectively.

For measurement using a reagent containing trans-aconitic acid as tricarboxylic acid or tricarboxylate, an apparatus having the same arrangement as described in Example 3 and the same measurement conditions as described in Example 3 were used. The same measurement method and method for processing measured data as described in Example 3 were used, except that the antigen solution was different.

The final concentrations of antibody and human CRP in a reaction system were about 0.036 mg/ml and the concentration of the human CRP solution used in measurement multiplied by 0.046, respectively.

Results are shown below. Figure **8** shows plots representing results of measurement in which a reagent contained citric acid as tricarboxylic acid or tricarboxylate and the human CRP solution having each concentration up to 100 mg/dl was added to each buffer solution. The vertical axis represents the intensity of scattered light, while the horizontal axis represents the concentration of the human CRP solution used for measurement. The plotted value was obtained by subtracting a measurement value (0 mg/dl), obtained when a buffer solution did not contain human CRP, from a measurement value of the human CRP solution having each concentration with respect to the same buffer solution. The temperature in a cell during measurement, which was measured with the thermocouple, was held at 25.5±1°C.

According to Figure **8**, when the comparative example, the MOPS buffer solution, was used to measure an antigen-antibody reaction, a sufficient difference in measurement value was not obtained in measurement of the CRP solution up to 20 mg/dl. In this case, substantially no difference in human CRP concentration could be detected (indicated by unfilled circles in Figure **8**). Even when measurement values were at least 30 mg/dl, differences between each measurement value were small and the resolution of the human CRP concentration was low. On the other hand, when measurement was performed using a reagent containing citric acid, higher measurement values were clearly indicated in measurement of the human CRP solution having each concentration. In this case, even in measurement of the human CRP solution up to 20 mg/dl, differences in human CRP concentration could be detected (indicated by filled circles in Figure **8**).

Figure **9** shows plots representing results of measurement in which a reagent contained trans-aconitic acid as tricarboxylic acid or tricarboxylate and the human CRP solution having each concentration up to 100 mg/dl was added to each buffer solution. The vertical axis represents a voltage value, while the horizontal axis represents the concentration of the human CRP solution used for measurement. The graph is assessed in a manner as in Figure 1. The plotted value was obtained by subtracting a measurement value (0 mg/dl), obtained when a buffer solution did not contain human CRP, from a measurement value of the human CRP solution having each concentration with respect to the same buffer solution.

According to Figure **9**, when the comparative example, the MOPS buffer solution, was used to measure an antigen-antibody reaction, no sufficient difference in measurement value was obtained in measurement of the CRP solution up to 10 mg/dl (indicated by unfilled circles in Figure **9**). On the other hand, when measurement was performed using a reagent containing trans-aconitic acid, higher measurement values were clearly indicated in measurement of the human CRP solution having each concentration. In this case, even in measurement of the human CRP solution up to 10 mg/dl, a difference in human CRP concentration could be detected (indicated by filled circles in Figure **9**).

As shown in Examples 7 and 8 described above, it could be confirmed that the immune reaction measurement method of the present invention can improve measurement values even in human CRP measurement.

### INDUSTRIAL APPLICABILITY

As described above, the present invention can provide an immune reaction measurement method which is capable of easily increasing measurement values. Further, the present invention can provide an immune reaction measurement method, which is capable of relaxing a zone phenomenon generated in an antigen excess region.

## Claims

1. An immune reaction measurement method for measuring an antigen or an antibody as subject substances contained in a sample, comprising:
step A of mixing tricarboxylic acid or tricarboxylate, an antibody or an antigen capable of specifically binding to the subject substance as specifically binding substances and the sample; and
step B of detecting an agglutination complex generated by an antigen-antibody reaction between the subject substance and the specifically binding substance in a reaction system comprising the sample, the specifically binding substance and the tricarboxylic acid or the tricarboxylate composed by step A,
wherein the tricarboxylic acid or tricarboxylate concentration of the reaction system is no more than 0.2 M and the pH of the reaction system is 4.5 when the antigen-antibody reaction is carried out.

2. The immune reaction measurement method according to claim 1, wherein a buffer is additionally mixed in step A.

3. The immune reaction measurement method according to claims 1 or 2, wherein the tricarboxylic acid is citric acid or aconitic acid.

4. The immune reaction measurement method according to any of claims 1 to 3, wherein the reaction system contains 2 to 6 wt polyethylene glycol.

5. The immune reaction measurement method according to claim 1, wherein in step B, the agglutination complex is detected by measuring optical variations attributed to the agglutination complex.

6. The immune reaction measurement method according to any of claims 1 to 5, wherein the antigen holds a metal ion within the molecular structure thereof, and the antibody capable of specifically binding to the antigen is capable of specifically binding to the antigen without the metal ion when the metal ion is released from the antigen.

7. The immune reaction measurement method according to any of claims 1 to 6, wherein the antigen is a substance having a plurality of binding sites with respect to at least one antibody, and the antibody is a monoclonal antibody capable of binding the plurality of binding sites of the antigen.

8. The immune reaction measurement method according to any of claims 1 to 5, wherein the antigen is human albumin.

9. The immune reaction measurement method according to any of claims 1 to 7, wherein the antigen is human C-reactive protein.

## Patentansprüche

1. Immunreaktion-Messverfahren zum Messen eines Antigens oder eines Antikörpers als Versuchssubstanzen, die in einer Probe enthalten sind, das umfasst:
einen Schritt A des Mischens von Tricarbonsäure oder Tricarboxylat, eines Antikörpers oder eines Antigens, das sich speziell an die Versuchssubstanz als spezielle Bindungssubstanzen binden kann, und der Probe; und
einen Schritt B des Detektierens eine Agglutinationskomplexes, der erzeugt wird durch eine Antigen-Antikörper-Reaktion zwischen der Versuchssubstanz und der speziell bindenden Substanz in einem Reaktionssystem, das die Probe, die speziell bindende Substanz und die Tricarbonsäure oder das Tricarboxylat enthält und im Schritt A gebildet worden ist,
wobei die Tricarbonsäuren- oder Tricarboxylat-Konzentration des Reaktionssystems nicht höher als 0,2 M ist und der pH-Wert des Reaktionssystems 4,5 beträgt, wenn die Antigen-Antikörper-Reaktion ausgeführt wird.

2. Immunreaktion-Messverfahren nach Anspruch 1, bei dem im Schritt A zusätzlich ein Puffer hinzugemischt wird.

3. Immunreaktion-Messverfahren nach den Ansprüchen 1 oder 2, bei dem die Tricarbonsäure Zitronensäure oder Akonitsäure ist.

4. Immunreaktion-Messverfahren nach einem der Ansprüche 1 bis 3, bei dem das Reaktionssystem 2 bis 6 Gew.-% Polyethylen-Glycol enthält.

5. Immunreaktion-Messverfahren nach Anspruch 1, bei dem im Schritt B der Agglutinationskomplex durch Messen optischer Änderungen, die dem Agglutinationskomplex zugeschrieben werden, detektiert wird.

6. Immunreaktion-Messverfahren nach einem der Ansprüche 1 bis 5, bei dem das Antigen ein Metallion in seiner molekularen Struktur hält und der Antikörper, der sich an das Antigen speziell binden kann, sich an das Antigen ohne das Metallion speziell binden kann, wenn das Metallion von dem Antigen freigegeben ist.

7. Immunreaktion-Messverfahren nach einem der Ansprüche 1 bis 6, bei dem das Antigen eine Substanz ist, die mehrere Bindungsstellen in Bezug auf wenigstens einen Antikörper besitzt, und der Antikörper ein monoklonaler Antikörper ist, der mehrere Bindungsstellen des Antigens binden kann.

8. Immunreaktion-Messverfahren nach einem der Ansprüche 1 bis 5, bei dem das Antigen menschliches Albumin ist.

9. Immunreaktion-Messverfahren nach einem der Ansprüche 1 bis 7, bei dem das Antigen menschliches C-reaktives Protein ist.

## Revendications

1. Procédé de mesure par immunoréaction destiné à mesurer un antigène ou un anticorps en tant que substances cibles contenues dans un échantillon, comprenant :
une étape A de mélange d'un acide tricarboxylique ou d'un tricarboxylate, d'un anticorps ou d'un antigène capable de se lier spécifiquement à la substance cible en tant que substances à liaison spécifique et de l'échantillon, et
une étape B de détection d'un complexe d'agglutination généré par une réaction antigène-anticorps entre la substance cible et la substance à liaison spécifique dans un système réactionnel comprenant l'échantillon, la substance à liaison spécifique et l'acide tricarboxylique ou bien le tricarboxylate constitué par l'étape A,
dans lequel la concentration de l'acide tricarboxylique ou du tricarboxylate du système réactionnel ne dépasse pas 0,2 M et où le pH du système réactionnel est de 4,5 lorsque la réaction antigène-anticorps est exécutée.

2. Procédé de mesure par immunoréaction selon la revendication 1, dans lequel un tampon est en outre mélangé à l'étape A.

3. Procédé de mesure par immunoréaction selon la revendication 1 ou 2, dans lequel l'acide tricarboxylique est l'acide citrique ou l'acide aconitique.

4. Procédé de mesure par immunoréaction selon l'une quelconque des revendications 1 à 3, dans lequel le système réactionnel contient de 2 à 6 % en poids de polyéthylène glycol.

5. Procédé de mesure par immunoréaction selon la revendication 1, dans lequel au cours de l'étape B, le complexe d'agglutination et détecté en mesurant les modifications optiques attribuées au complexe d'agglutination.

6. Procédé de mesure par immunoréaction selon l'une quelconque des revendications 1 à 5, dans lequel l'antigène contient un ion métallique dans sa structure moléculaire, et l'anticorps capable d'une liaison spécifique à l'antigène peut se lier spécifiquement à l'antigène sans l'ion métallique lorsque l'ion métallique est libéré de l'antigène.

7. Procédé de mesure par immunoréaction selon l'une quelconque des revendications 1 à 6, dans lequel l'antigène est une substance comportant une pluralité de sites de liaison en ce qui concerne au moins un anticorps, et l'anticorps est un anticorps monoclonal capable de se lier à une pluralité de sites de liaison de l'antigène.

8. Procédé de mesure par immunoréaction selon l'une quelconque des revendications 1 à 5, dans lequel l'antigène est de l'albumine humaine.

9. Procédé de mesure par immunoréaction selon l'une quelconque des revendications 1 à 7, dans lequel l'antigène est une protéine C-réactive humaine.
